# EUROPEAN PATENT SPECIFICATION

(11) **EP 4 547 324 B1**
(45) Date of publication and mention of the grant of the patent: **11.02.2026**
(21) Application number: 23744562.2
(22) Date of filing: 30.06.2023
(51) Int. Cl.: A61N 1/372, A61N 1/375, A61M 25/00, A61M 25/01

(54) **BIOSTIMULATOR TRANSPORT SYSTEM HAVING FLEXIBLE TETHER**
BIOSTIMULATORTRANSPORTSYSTEM MIT FLEXIBLEM HALTEGURT
SYSTÈME DE TRANSPORT DE BIOSTIMULATEUR À ATTACHE FLEXIBLE

(30) Priority: 01.07.2022 US 202263358061 P; 27.06.2023 US 202318215091
(43) Date of publication of application: 07.05.2025
(73) Proprietor: Pacesetter, Inc., Sylmar, CA 91342 (US)
(72) Inventor: ARNAR, Bernhard, Sylmar, California 91342 (US); BLUE, Jeremiah, Sylmar, California 91342 (US); WEBER, Adam, Sylmar, California 91342 (US); GULSETH, Jared, Sylmar, California 91342 (US); SACHA, Mike, Sylmar, California 91342 (US)
(74) Representative: Barker Brettell Sweden AB
(86) International application number: PCT/IB2023/056859
(87) International publication number: WO 2024/003866

(56) References cited:
- US-A1- 2015 273 207
- US-A1- 2016 243 355
- US-A1- 2018 178 006
- US-A1- 2020 253 614

## Description

### BACKGROUND

### FIELD

The present disclosure relates to biostimulator transport systems and methods. More specifically, the present disclosure relates to transport systems for delivery or retrieval of leadless biostimulators.

### BACKGROUND INFORMATION

Cardiac pacing by an artificial pacemaker provides an electrical stimulation to the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Cardiac pacing by currently available or conventional pacemakers is usually performed by a pulse generator implanted subcutaneously or sub-muscularly in or near a patient's pectoral region. The generator usually connects to a proximal end of one or more implanted leads, the distal end of which contains one or more electrodes for positioning adjacent to the inside or outside wall of a cardiac chamber. Although more than one hundred thousand conventional cardiac pacing systems are implanted annually, various well-known difficulties exist, of which a few will be cited. For example, a pulse generator, when located subcutaneously, presents a bulge in the skin that patients can find unsightly, unpleasant, or irritating, and which patients can subconsciously or obsessively manipulate or "twiddle." Even without persistent manipulation, subcutaneous pulse generators can exhibit erosion, extrusion, infection, disconnection, insulation damage, or conductor breakage at the wire leads. Although sub-muscular or abdominal placement can address some concerns, such placement involves a more difficult surgical procedure for implantation and adjustment, which can prolong patient recovery.

Leadless cardiac pacemakers incorporate electronic circuitry at the pacing site and eliminate leads, and thus, avoid the above-mentioned shortcomings of conventional cardiac pacing systems. Leadless cardiac pacemakers can be anchored at the pacing site by an anchor. During delivery or retrieval of a leadless cardiac pacemaker, a transport system can apply torque to the leadless cardiac pacemaker via a docking cap to screw the anchor into, or out of, the target tissue.

US 2018/1788006 A1 discloses delivery devices, systems, and methods for delivering implantable leadless pacing devices. The delivery device includes an intermediate tubular member and an inner tubular member slidably disposed within a lumen of the intermediate tubular member. A distal holding section extends distally of a distal end of the intermediate tubular member and defines a cavity therein for receiving an implantable leadless pacing device. At least a portion of the lumen of the inner tubular member is bifurcated to form a first lumen and a second lumen. The first lumen is configured to receive a first portion of a tether and the second lumen is configured to receive a second portion of the tether

### SUMMARY

Existing biostimulator transport systems used for delivery or retrieval of leadless cardiac pacemakers may have a set of tethers that include end features to engage an attachment feature of a leadless biostimulator. The tethers can be moved relative to each other to align or misalign the end features. In the aligned state, the end features can lock the tethers to the leadless biostimulator. In the misaligned state, the end features can unlock the tethers from the leadless biostimulator. Accordingly, adjustment of the end features can retain or release the leadless biostimulator.

The tethers, end features, and accompanying mechanisms of existing biostimulator transport systems can be complex and expensive. More particularly, the mechanisms and components used to implement the biostimulator retention/release feature of existing biostimulator transport systems may require precise movements and fine mechanical tolerances. Furthermore, the cyclic loading seen by the biostimulator transport system within the target anatomy can challenge the precise movements and complicate delivery of the leadless biostimulator. Accordingly, biostimulator transport systems would benefit from mechanisms of retention and release that are simple, inexpensive, and reliable.

A biostimulator transport system, such as a catheter-based system for delivering or retrieving a leadless cardiac pacemaker, having a flexible tethering mechanism, is provided. According to the invention, which is defined by claim 1, the biostimulator transport system includes a handle including a housing and a knob releasably coupled to the housing. A torque shaft extends longitudinally distal of the handle, and a docking cap is coupled to the torque shaft. The docking cap has a docking cavity to receive an attachment feature of a biostimulator. A tether support extends through the torque shaft to a distal end. The tether support has one or more support lumens to receive a tether. More particularly, the tether extends through the one or more support lumens to a tether bight distal to the distal support end. The tether is coupled to the knob. The tether bight can extend through the attachment feature to retain the biostimulator. The tether can reliably retain and release the biostimulator.

The tether can have several tether legs extending from the tether bight. For example, the tether legs can extend proximally through support lumens in the tether support. The tether and, thus, the tether legs can be polymeric filaments, e.g., an ultra-high molecular weight polyethylene thread or polyester thread, or metallic filaments. The filament can be inexpensive and, thus, can provide an economical mode of retaining and releasing the biostimulator.

According to the invention, the handle includes the housing, and the knob can be releasably coupled to the housing. The tether can be coupled to one or more of the housing or the knob. For example, a first tether leg can be coupled to the knob and a second tether leg can be coupled to the housing. The second tether leg can be exposed through a window in the housing such that a user can sever the second tether leg through the window. The knob may then be retracted from the housing to remove the tether from the attachment feature and release the biostimulator.

In an embodiment, the knob can be rotatably coupled to the housing. The knob can include a blade positioned such that rotation of the knob causes the blade to cut the second tether leg. The knob can then be retracted to remove the tether from the attachment feature and release the biostimulator.

Other release mechanisms are described for removing the tether from the attachment feature to release the biostimulator. For example, a rod can be releasably coupled to the second tether leg. Releasing the second tether leg from the rod can release the biostimulator.
Alternatively, the biostimulator transport system can include a snare extending through the support lumen. The snare may include several snare loops.

Embodiments include an attachment feature for a leadless biostimulator, which can engage the tether. The attachment feature can include a base and a button. The button can have an insertion slot and a retention slot. The insertion slot can receive the tether, and the tether may be subsequently secured in the retention slot. Accordingly, the retention slot can extend from the insertion slot, e.g., in a direction transverse to a direction that the insertion slot extends into the button. The insertion slot can be vertically directed or horizontally directed. The insertion slot may include a non-linear profile, e.g., a w-shaped profile.

The above summary does not include an exhaustive list of all aspects of the present invention. It is contemplated that the invention is defined by the appended claims only and may include all systems that can be practiced from all suitable combinations of the various aspects summarized above, as well as those disclosed in the Detailed Description below. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a diagrammatic medial-lateral cross section of a patient heart illustrating an example implantation of biostimulators in the patient heart, in accordance with an embodiment.
FIG. 2 is a side view of a biostimulator, in accordance with an embodiment.
FIG. 3 is a perspective view of a biostimulator transport system, in accordance with an embodiment.
FIG. 4 is a side view of a distal portion of a biostimulator transport system in a docked configuration, in accordance with an embodiment.
FIG. 5 is a side view of a distal portion of a biostimulator transport system in an undocked configuration, in accordance with an embodiment.
FIG. 6 is a perspective view of a distal portion of a biostimulator transport system in an undocked configuration, in accordance with an embodiment.
FIG. 7 is a perspective view of a tether holding an attachment feature, in accordance with an embodiment.
FIG. 8 is a perspective view of a distal portion of a biostimulator transport system in an undocked configuration, in accordance with an embodiment.
FIG. 9 is a perspective view of a distal portion of a tether support, in accordance with an embodiment.
FIG. 10 is a perspective view of a handle of a biostimulator transport system having a cutting window, in accordance with an embodiment.
FIGS. 11-12 are perspective views of a handle of a biostimulator transport system having a cutting wheel, in accordance with an embodiment.
FIG. 13 is a perspective view of a handle of a biostimulator transport system having a vise, in accordance with an embodiment.
FIG. 14 is a perspective view of a handle of a biostimulator transport system having an open shroud, in accordance with an embodiment.
FIG. 15 is a perspective view of a handle of a biostimulator transport system having a collet, in accordance with an embodiment.
FIGS. 16-17 are perspective views of a handle of a biostimulator transport system having a twist release, in accordance with an embodiment.
FIGS. 18-19 are pictorial views of a loop and rod mechanism of a biostimulator transport system, in accordance with an embodiment.
FIGS. 20-21 are pictorial views of a loop and hook mechanism of a biostimulator transport system, in accordance with an embodiment.
FIGS. 22-23 are pictorial views of a loop and clasp mechanism of a biostimulator transport system, in accordance with an embodiment.
FIGS. 24-25 are pictorial views of a snare mechanism of a biostimulator transport system, in accordance with an embodiment.
FIG. 26 is a pictorial view of a loading mechanism for a biostimulator transport system, in accordance with an embodiment.
FIGS. 27-29 are pictorial views of a twist-to-load attachment feature of a leadless biostimulator, in accordance with an embodiment.
FIGS. 30-32 are pictorial views of a w-slot attachment feature of a leadless biostimulator, in accordance with an embodiment.
FIGS. 33-35 are pictorial views of an undercut-slot attachment feature of a leadless biostimulator, in accordance with an embodiment.

### DETAILED DESCRIPTION

Embodiments describe a biostimulator transport system, e.g., a catheter-based system for delivery or retrieval of a leadless cardiac pacemaker, having a flexible tether. The biostimulator may be used to pace cardiac tissue as described below. The biostimulator, however, may be used in other applications, such as deep brain stimulation, and thus, reference to the biostimulator as being a cardiac pacemaker is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator transport system is provided. The biostimulator transport system includes a tether, e.g., a fibrous tether, to retain a biostimulator. The tether can have a loop that connects the biostimulator to the biostimulator transport system. The biostimulator transport system can include a mechanism to disengage the tether. For example, a handle of the biostimulator transport system can include a cutter, a vise, or another mechanism to decouple one tether leg from the handle. Another leg of the tether can be connected to a knob. The knob can be removed from the handle to remove the tether from the biostimulator. Accordingly, the biostimulator transport system can provide a simple, inexpensive, and reliable mechanism to retain and release the biostimulator.

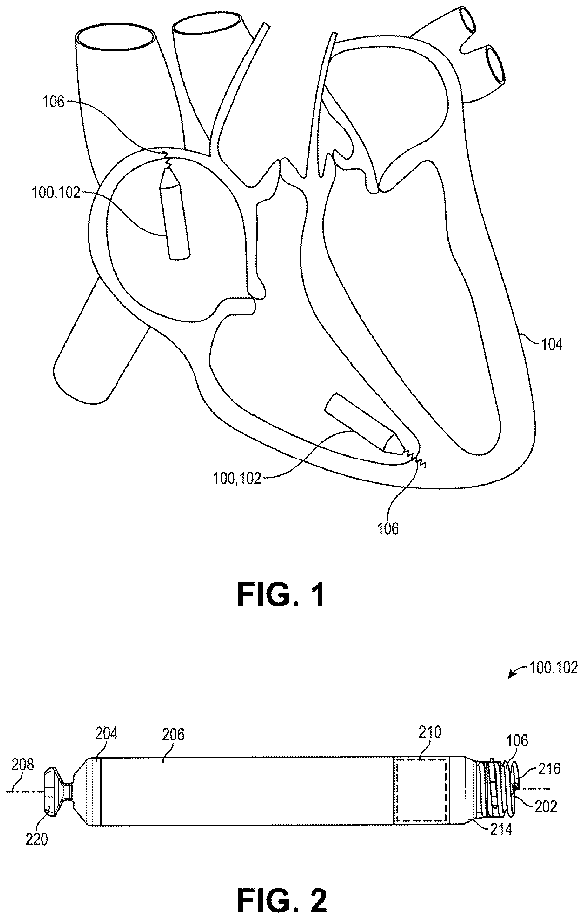

Referring to FIG. 1, a diagrammatic medial-lateral cross section of a patient heart illustrating an example implantation of biostimulators in the patient heart is shown in accordance with an embodiment. A cardiac pacing system includes one or more biostimulators 100. The biostimulators 100 can be implanted in the patient heart 104, and can be leadless, and thus may be leadless cardiac pacemakers 102. Each biostimulator 100 can be placed in a cardiac chamber, such as a right atrium and/or a right ventricle of the patient heart 104, or attached to an inside or outside of the cardiac chamber. Attachment of the biostimulator 100 to the cardiac tissue can be accomplished via one or more fixation elements 106, such as helical anchors. In a particular embodiment, the leadless cardiac pacemaker 102 can use two or more electrodes located on or within a housing of the leadless cardiac pacemaker 102 for pacing the cardiac chamber upon receiving a triggering signal from internal circuitry and/or from at least one other device within the body.

Referring to FIG. 2, a side view of a biostimulator, in accordance with an embodiment. The biostimulator 100 can be a leadless cardiac pacemaker 102 that can perform cardiac pacing and that has many of the advantages of conventional cardiac pacemakers while extending performance, functionality, and operating characteristics. The biostimulator 100 can have two or more electrodes, e.g., a distal electrode 202 and a proximal electrode 204, located within, on, or near a biostimulator housing 206 of the biostimulator 100. In an embodiment, one or more of the fixation elements 106 forms a portion of the distal electrode 202. The electrodes can deliver pacing pulses to muscle of the cardiac chamber, and optionally, can sense electrical activity from the muscle. The electrodes may also communicate bidirectionally with at least one other device within or outside the body.

In an embodiment, the biostimulator housing 206 has a longitudinal axis 208, and the distal electrode 202 can be a distal pacing electrode mounted on the biostimulator housing 206 along the longitudinal axis 208. The biostimulator housing 206 can contain a primary battery to provide power for pacing, sensing, and communication, which may include, for example bidirectional communication. The biostimulator housing 206 can optionally contain an electronics compartment 210 to hold circuitry adapted for different functionality. For example, the electronics compartment 210 can contain circuits for sensing cardiac activity from the electrodes, circuits for receiving information from at least one other device via the electrodes, circuits for generating pacing pulses for delivery via the electrodes, or other circuitry. The electronics compartment 210 may contain circuits for transmitting information to at least one other device via the electrodes and can optionally contain circuits for monitoring device health. The circuit of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

Leadless pacemakers or other leadless biostimulators 100 can be fixed to an intracardial implant site by one or more actively engaging mechanism or fixation mechanism. The fixation mechanism can include a screw or helical member that screws into the myocardium. Alternatively, the fixation mechanism can include tines that pierce and grip the myocardium.

In an embodiment, the biostimulator 100 includes the fixation element 106 coupled to the biostimulator housing 206. The fixation element 106 can be a helical element to screw into target tissue. More particularly, the fixation element 106 can extend helically from a flange 214 of the biostimulator 100, which is mounted on the biostimulator housing 206, to a distal tip at a helix distal end 216.

The helix distal end 216 can be located distal to the distal electrode 202 (a centrally located electrode). Accordingly, when the biostimulator 100 contacts the target tissue, the distal tip can pierce the tissue and the housing 206 can be rotated to screw the outer fixation element 106 into the target tissue to pull the distal electrode 202 into contact with the tissue.

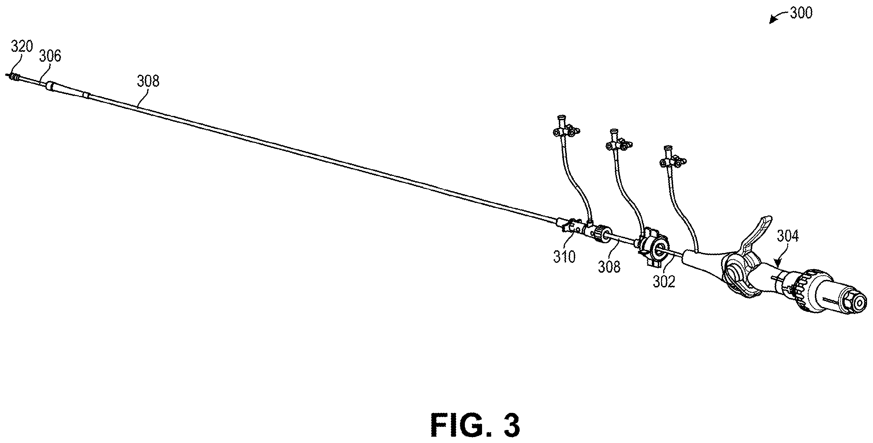

The biostimulator 100 includes an attachment feature 220. The attachment feature 220 generally facilitates coupling of the biostimulator to a biostimulator transport system (FIG. 3). More particularly, the attachment feature can include features that can be engaged and retained by a tether of the biostimulator transport system.

Referring to FIG. 3, a perspective view of a biostimulator transport system is shown in accordance with an embodiment. Leadless pacemakers or other leadless biostimulators 100 can be delivered to and retrieved from a patient using a biostimulator transport system 300, as described below. In some implementations, the biostimulator transport system 300 is a delivery system for delivering the leadless pacemaker to the target tissue. In some implementations, the biostimulator transport system 300 is a retrieval system for retrieving the leadless pacemaker from the target tissue.

The biostimulator transport system 300 can include an elongated catheter 302 extending distally from a handle 304 to a distal catheter end 306. The elongated catheter 302 can be a deflectable catheter, and an operator can use the handle 304 to steer the distal catheter end 306 in the patient. In an embodiment, the biostimulator transport system 300 includes a guide catheter 308 mounted on the elongated catheter 302. The guide catheter 308 can be slidably disposed on the elongated catheter 302 such that a distal portion of the guide catheter 308 can slide distally over the distal catheter end 306 of the elongated catheter 302 and/or the biostimulator 100. Similarly, the biostimulator transport system 300 can include an introducer hub assembly 310 mounted on the guide catheter 308. The introducer hub assembly 310 can be slidably disposed on the guide catheter 308 such that a distal portion of the introducer hub assembly 310 can slide distally over the distal catheter end 306 of the elongated catheter 302 and/or the distal portion of the guide catheter 308. More particularly, the introducer hub assembly 310 can be inserted into an access sheath to gain access to the patient vasculature, and after access is established, the distal portion of the guide catheter 308 and/or the distal catheter end 306 of the elongated catheter 302 can be advanced through the access sheath into the patient.

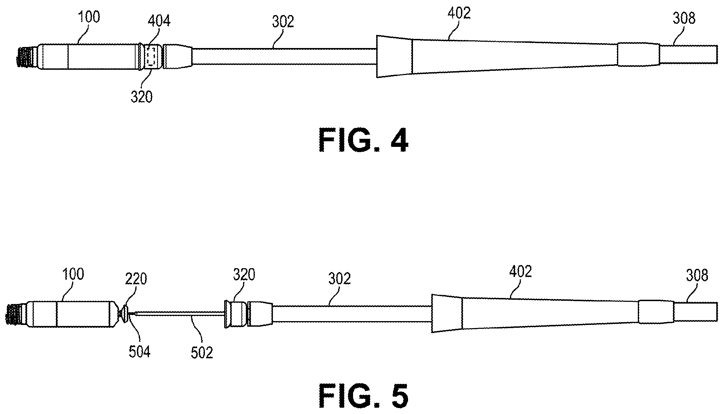

The distal catheter end 306 of the elongated catheter 302 may be selectively connectable to the biostimulator 100. More particularly, the biostimulator 100 can be mounted on the distal catheter end 306 of the elongated catheter 302. The biostimulator 100 can be protected by a protective sheath (FIGS. 4-5) of the distal portion of the guide catheter 308 during delivery and/or retrieval of the biostimulator 100 from the patient. Accordingly, the biostimulator 100 can be advanced into the patient along with the distal catheter end 306.

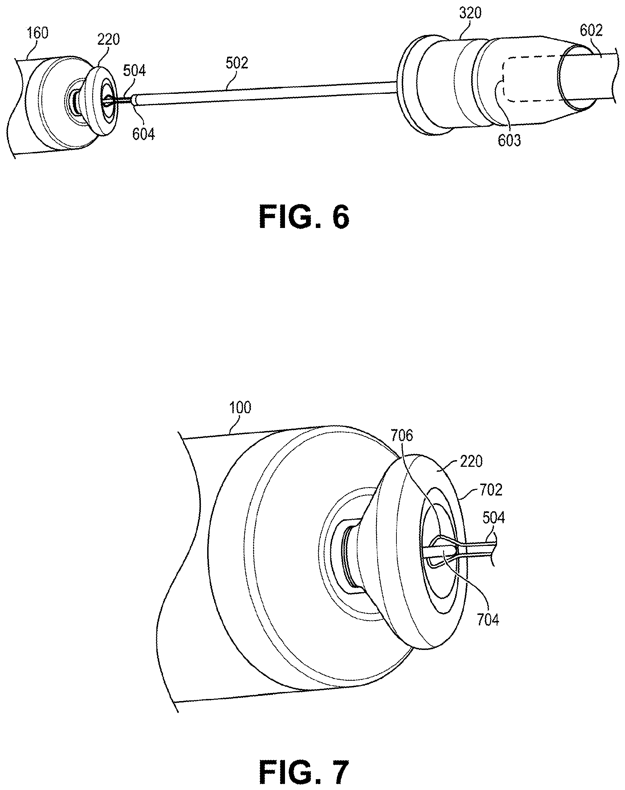

The leadless pacemaker system can be used to implant one or more biostimulators 100 within an atrium and/or a ventricle of a heart 104 of the patient. Implantation of each biostimulator 100 may be achieved, in part, by endocardial insertion of the biostimulators 100. For example, the elongated catheter 302 of the leadless pacemaker system can include a torque shaft (FIG. 6) coupled to a docking cap 320. The docking cap 320 can have a docking cavity (FIG. 4) to receive the attachment feature 220 of the biostimulator 100. The torque shaft can be torqueable and rotation of the torque shaft can rotate the docking cap 320, which can impart rotation to the attachment feature 220. Accordingly, torque can be transmitted through the torque shaft to rotate the biostimulator 100 in a first direction, e.g., clockwise. Rotating the biostimulator 100 when the fixation element 106 is in contact with the heart tissue can cause the fixation element 106 to screw into the heart tissue and affix the biostimulator 100 to the heart tissue. Similarly, removal and retrieval of the biostimulators 100 may be accomplished endocardially. For example, the torque shaft of the elongated catheter 302 can be rotated in a second direction, e.g., counterclockwise, to transmit torque through the docking cap 320 to the attachment feature 220 to disengage the biostimulator 100 from the heart tissue. Accordingly, delivery and retrieval systems having a structure similar to that shown in FIG. 3 may be used to deliver and/or retrieve the biostimulator 100 from a target anatomy.

Referring to FIG. 4, a side view of a distal portion of a biostimulator transport system in a docked configuration is shown in accordance with an embodiment. A distal portion of the guide catheter 308 can include a protective sheath 402 that flairs radially outward in a distal direction. Guide catheter 308 can be movable in the longitudinal direction relative to the elongated catheter 302, and thus, the protective sheath 402 may be retracted to expose the docking cap 320 at the distal end of the elongated catheter 302.

In an embodiment, the docking cap 320 has a docking cavity 404 to receive a portion of the biostimulator 100. More particularly, the docking cavity 404 can receive the attachment feature 220 of the biostimulator 100 when the biostimulator is in a docked state relative to the biostimulator transport system 300. In the docked state, rotation of the docking cap 320 can translate to rotation of the biostimulator 100 via engagement with the attachment feature 220.

Referring to FIG. 5, a side view of a distal portion of a biostimulator transport system in an undocked configuration is shown in accordance with an embodiment. The biostimulator 100 may be moved to an undocked state relative to the biostimulator transport system 300. More particularly, the elongated catheter 302 can be moved proximally relative to the biostimulator 100 to retract the docking cap 320 and move the attachment feature 220 out of the docking cavity 404.

In an embodiment, when the biostimulator 100 is in the undocked state, the biostimulator 100 remains connected to the biostimulator transport system 300 through a tethering subsystem. The tethering subsystem can include a tether support 502 extending longitudinally from the elongated catheter 302 toward the attachment feature 220 of the biostimulator 100. The tether subsystem can also include a tether 504 extending through the tether support 502 to the attachment feature 220. More particularly, the tether 504 can engage the attachment feature 220, and the tether support 502 can extend around the tether 504 to provide column strength to the tether 504 while the biostimulator 100 is disengage from the docking cap 320. In the undocked state, the biostimulator 100 can be rotatably decoupled from the docking cap 320, while remaining tethered to the biostimulator transport system 300.

Referring to FIG. 6, a perspective view of a distal portion of a biostimulator transport system in an undocked configuration is shown in accordance with an embodiment. In the perspective view, the elongated catheter 302 is hidden to expose and illustrate a torque shaft 602 having a distal shaft end 603. The distal shaft end 603 can be coupled to the docking cap 320. Accordingly, the torque shaft 602 can be rotated to transmit torque to the docking cap 320 (and to the biostimulator 100 in the docked state).

The torque shaft 602 can include a shaft lumen, and the tether support 502 can extend through the shaft lumen to a distal support end 604. Similarly, the tether 504 can extend through a lumen of the tether support 502. The tether 504 can engage the attachment feature 220 of the biostimulator 100. The tether support 502 can be axially movable relative to the tether 504. Accordingly, the distal support end 604 can be advanced nearer to the attachment feature 220 to reduce an exposed length of the tether 504. By contrast, the distal support end 604 can be retracted farther from the attachment feature 220 to increase the exposed length of the tether 504. The tether 504 may be less stiff than the tether support 502, and thus, when more tether 504 is exposed, the biostimulator motion is more decoupled from the effect of the biostimulator transport system 300.

Referring to FIG. 7, a perspective view of a tether holding an attachment feature is shown in accordance with an embodiment. A close-up view of the attachment feature 220 indicates that the attachment feature 220 can include a recess in a proximal end of a button 702 of the attachment feature 220. The button 702 can be a proximal portion of the attachment feature 220 that engages corresponding features within the docking cap 320. More particularly, the button 702 can engage keys of the docking cap 320 such that rotation of the docking cap 320 can cause the keys to engage the button 702 and transmit torque through the button 702 to the biostimulator 100.

A cross beam 704, post, etc., can extend over the recess. A gap may therefore be formed between the cross beam 704 and an inner surface of the recess. In embodiment, the tether 504 includes a tether bight 706 that loops around the cross beam 704. More particularly, the tether 504 can extend through the gap and loop around the cross beam 704 to form the tether bight 706. The tether bight 706 can be disposed within the gap distal to the distal support end 604 of the tether support 502.

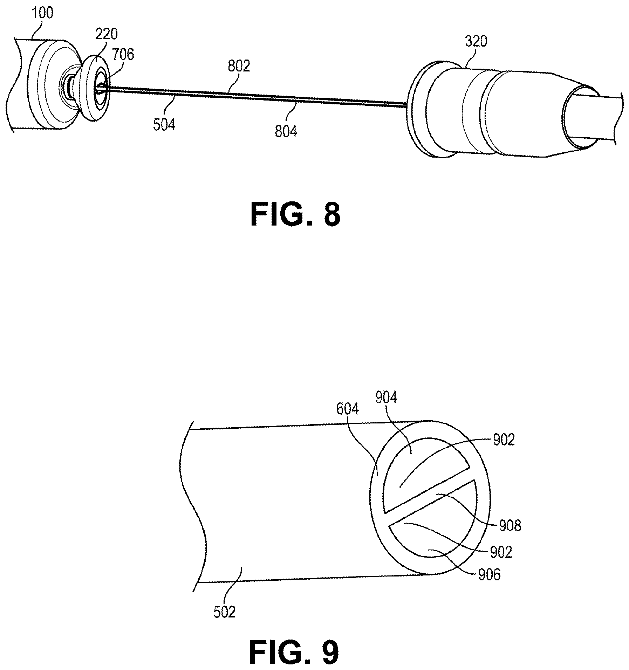

Referring to FIG. 8, a perspective view of a distal portion of a biostimulator transport system in an undocked configuration is shown in accordance with an embodiment. The biostimulator transport system 300 can be transitioned into a tether mode. For example, after the biostimulator 100 has been affixed to the target pacing site, the tether support 502 can be retracted to expose more of the tether 504. When the system is in the tether mode, the biostimulator 100 can emulate the motion that it will undergo when it is freely affixed to the target tissue. More particularly, the biostimulator 100 can behave as though it is disconnected from the biostimulator transport system 300. Such behavior is possible because the tether 504 can be formed from a flexible material, as described below, which does not impede motion of the biostimulator 100. In the tether mode, an integrity of the fixation of the biostimulator 100 to the target tissue can be assessed. Furthermore, the electrical performance of the biostimulator 100 can be evaluated, e.g., a threshold voltage (a minimum voltage required to transmit a pacing pulse to the target tissue), an impedance, and a current of injury can be assessed while the biostimulator 100 is in the tether mode prior to release of the biostimulator 100 from the biostimulator transport system 300.

In the tether mode, the biostimulator 100 may be connected to the biostimulator transport system 300 by several lengths of the flexible tether 504. More particularly, the tether 504 can include a first tether leg 802 extending proximally from the tether bight 706, and a second tether leg 804 extending proximally from the tether bight 706. The tether legs can extend adjacent to each other longitudinally between the attachment feature 220 and the docking cap 320. The tether legs can be flexible, and thus the biostimulator 100 is not rigidly attached to the biostimulator transport system 300. That is, the biostimulator 100 may freely move in the tether mode without being impeded by the attached tether. The ends of the tether legs may, however, extend through the elongated catheter 302 and be attached to the handle 304. Thus, the tether legs can physically connect the biostimulator 100 to the biostimulator transport system 300 while decoupling movement of the biostimulator 100 from the biostimulator transport system 300.

Referring to FIG. 9, a perspective view of a distal portion of a tether support is shown in accordance with an embodiment. The flexibility of the tether 504 may be advantageous during the tether mode, however, under other circumstances there may be a need to support the tethers 504. For example, it may be necessary to apply forward pressure to the biostimulator 100 in the undocked state, and given that the tethers 504 are flexible and cannot push on the biostimulator 100, the tether support 502 may be advanced over the tether 504 to apply forward pressure to the button 702. Accordingly, the tether support 502 may have a structure that imparts column strength to the tether 504 and does not buckle when applying forward pressure to the biostimulator 100. The tether support 502 may, for example, be formed from a solid tube. The tubular structure can be formed from a polymer and may have sufficient stiffness to avoid buckling during use. Alternatively, the tether support 502 may be formed from a coil. The coil can include a metallic wire. The coil can provide good flexibility, however, the turns of the coil can stack on each other to also impart good column strength to the overall structure. Accordingly, the tether support 502 can be flexible enough to track through the anatomy and stiff enough to apply forward pressure on the biostimulator 100 when needed.

The flexibility of the tether legs can lead to the tether 504 becoming tangled under some circumstances. For example, when the torque shaft 602 and/or the docking cap 320 are rotated, torsion may be applied to the tethers 504 such that the tethers become twisted and tangled. Furthermore, cyclic motion of the tethers 504 when the tethers are exposed distally from the docking cap 320 may lead to twisting and/or tangling on the attachment feature 220. Tangling of the tethers 504 may impede release of the biostimulator 100 because the tangled tethers 504 may be unable to move freely and retract from the attachment feature 220. Accordingly, the support tube may have a structure that maintains a relative position between the tether legs to prevent tangling.

In an embodiment, the tether support 502 has one or more support lumens extending longitudinally within an outer support wall. For example, the one or more support lumens may be a single tubular lumen extending through the length of a flexible, metallic coil or a polymeric extrusion. Alternatively, the one or more support lumens 902 can include a first support lumen 904 separated from a second support lumen 906 by a support septum 908. For example, the tether support 502 may include a dual lumen polymeric extrusion. The support septum 908 can extend longitudinally along the length of the tether support 502 to form the first support lumen 904 radially separated from the second support lumen 906. The tether support 502 and the lumen(s) can terminate at the distal support end 604. Accordingly, the support lumen(s) can open to the surrounding environment at the distal support end 604.

As described above, the tether 504 can extend through the support lumen 902. For example, the tether legs can extend proximally from the tether bight 706 and pass into the openings at the distal support end 604 to track through the one or more support lumen(s). More particularly, the tether 504 can extend through the support lumen(s) to the tether bight 706. In the case of a single support lumen 902, both tether legs can pass from the handle 304 through the support lumen 902 to the tether bight 706. When the tether support 502 has a multi-lumen structure, however, the first tether leg 802 can extend proximally from the tether bight 706 through the first support lumen 904, and the second tether leg 804 can extend proximally from the tether bight 706 through the second support lumen 906. In either case, the tether support 502 can provide column strength to the tether 504. Furthermore, the support lumens 902 can reduce the likelihood that the tether 504 will become tangled on and/or entangled with the button 702. For example, the support septum 908 can maintain the tether legs in a radially separated configuration to reduce a likelihood that the legs will twist around themselves and/or button 702.

The tether 504 may have a variety of flexible configurations. For example, the tether 504 may be formed from a non-metallic material that is flexible. The non-metallic material can include a polymer. For example, the tether 504 may be formed from a suture material, such as one or more of ultra-high molecular weight polyethylene (UHMWPE) or polyester (PE). Alternatively, the tether 504 may incorporate materials such as Kevlar^{®}, Vectran^{™}, etc.

The tether 504 may be formed from fibers or filaments of the non-metallic material. For example, the tether 504 may have a monofilament construction. Alternatively, several fibers or filaments can be combined to form a continuous tether length. For example, the tether 504 can include polymeric filaments, such as UHMWPE or PE threads, that are woven, twisted, or otherwise combined to form the tether 504 having a length sufficient to pass from a first end at the handle 304, through the elongated catheter 302 and tether support 502, and through a loop back to a second end at the handle 304. The fibrous, polymeric tether 504 can have high tensile strength and a virtually infinite fatigue life, while also exhibiting suppleness and flexibility at low cost. In an embodiment, the tether 504 has at least a 5 pound-force test strength.

In an embodiment, the tether 504 is formed from a metallic material. More particularly, the tether 504 can include a metallic filament. The tether 504 can have a monofilament, e.g., wire, structure or a multi-filament, e.g., cable, structure. The metallic material may be selected from biocompatible metals such as stainless steel or nickel titanium. The metallic tether 504 can be flexible and have high tensile strength to perform as described herein.

The use of a flexible tether 504 to connect the biostimulator 100 to the biostimulator transport system 300 is described above. In an embodiment, to release the biostimulator 100 at the target tissue site, one of the tether legs is retracted to pull the tether 504 through the gap until the other tether leg is retracted away from the attachment feature 220, leaving the biostimulator 100 free from the tether 504 and the biostimulator transport system 300. During delivery, the tether legs can be connected to a portion of the biostimulator transport system 300, e.g., the handle 304, to secure the biostimulator 100. Accordingly, in order to release the biostimulator 100, the biostimulator transport system 300 can include mechanisms to allow the tether legs to be released and/or removed from the handle 304. Embodiments of such mechanisms are described below.

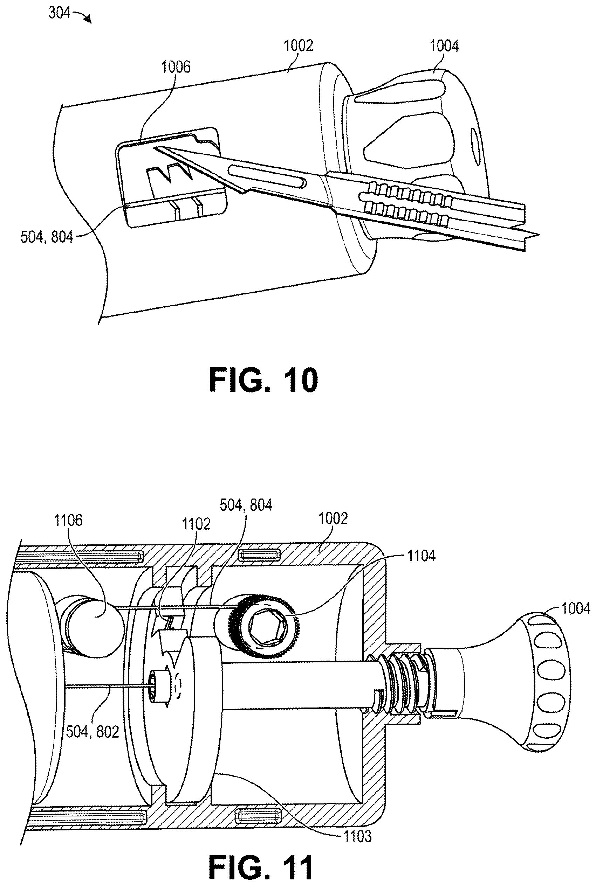

Referring to FIG. 10, a perspective view of a handle of a biostimulator transport system having a cutting window is shown in accordance with an embodiment. The handle 304 of the biostimulator transport system 300 can include a housing 1002. The housing 1002 can provide a shell that surrounds the internal components of the handle 304 and allows a user to grip and manipulate the handle 304. The handle 304 may also include a knob 1004 that is releasably coupled to the housing 1002. For example, the knob 1004 may be locked to the housing 1002 in a first configuration, and a user may rotate the knob 1004 to unlock the knob from the housing 1002. When unlocked, the user may pull on the knob 1004 to retract the knob from the housing 1002, and separate the knob from the handle 304. The knob 1004 may be locked to the housing 1002 by various mechanisms, including threads, keys, detents, etc. Accordingly, mechanisms for securing the knob 1004 to the handle 304 described herein are provided by way of example and not limitation.

In an embodiment, the first tether leg 802 (not shown) extends proximally from the tether bight 706 and is coupled to the knob 1004. For example, an end of the first tether leg 802 can be adhered to or tied onto the knob 1004. The first tether leg 802 may be attached such that, when the knob 1004 is retracted from the housing 1002, the first tether leg 802 is pulled along with the knob 1004. The second tether leg 804 of the tether 504, however, can extend proximally from the tether bight 706 to attach to the housing 1002. Accordingly, the knob 1004 may not be retracted when the second tether leg 804 is secured to the housing 1002, because the tether 504 will hold the knob 1004 and prevent its removal from the housing 1002.

The handle 304 can include a mechanism to cut, disconnect, or otherwise release the second tether leg 804 from the housing 1002 to allow the knob 1004 to be released and removed from the housing 1002. In an embodiment, the housing 1002 includes a window 1006, and the second tether leg 804 can be exposed through the window 1006 in the housing 1002. The window 1006 can be a cutaway section of a housing wall, and the section may be positioned laterally from and over the second tether leg 804 such that the window 1006 provides a line of sight from the surrounding environment to the second tether leg 804 within the housing 1002.

The second tether leg 804 may be accessible through the window 1006. More particularly, the user may insert a scalpel or another cutting device through the window 1006 toward the second tether leg 804. The user can cut the second tether leg 804 through the window 1006 to release the second tether leg 804 from the housing 1002. After the tether 504 is severed, the knob 1004 can be unlocked and pulled out of the housing 1002. The knob 1004 can be retracted until a severed end of the second tether leg 804 is pulled through the gap in the attachment feature 220, thereby releasing the biostimulator 100 from the tether 504. Accordingly, a process of releasing the biostimulator 100 can include disconnecting one end of the tether 504 from the housing 1002, and pulling on another end of the tether 504 until the tether is disconnected from the attachment feature 220.

Referring to FIG. 11, a perspective view of a handle of a biostimulator transport system having a cutting wheel is shown in accordance with an embodiment. Various mechanisms for achieving the release process described above are contemplated. In an embodiment, the knob 1004 is rotatably coupled to the housing 1002. For example, the knob 1004 can be threadably engaged to the housing 1002. The knob 1004 may be operably connected to a blade 1102 within the housing 1002. The blade 1102 may be part of a cutting wheel 1103 that is rotatably coupled to the housing 1002. More particularly, the cutting wheel 1103 may be longitudinally constrained and rotationally free within the housing 1002. The cutting wheel 1103 can have a keyway that is engaged by a distal end of the knob 1004. Accordingly, the blade 1102 may be mounted on the knob 1004 via the cutting wheel 1103 such that rotation of the knob 1004 causes the blade 1102 to move or rotate within the housing 1002.

As described above, the first tether leg 802 can be connected to the knob 1004. By contrast, the second tether leg 804 may connect to the housing 1002. For example, the second tether leg 804 can be secured to an attachment point on the housing 1002, e.g., by a screw 1104, such as a socket head cap screw. The second tether leg 804 can extend distally from the attachment point and be wrapped around the post 1106. When the tether 504, e.g., second tether leg 804, is wrapped around the post 1106, the clamping force required to secure the tether 504 at the attachment point can be reduced. The length of the tether 504 between the attachment point and the post 1106 may be aligned with the blade 1102. More particularly, rotation of the cutting wheel 1103 can cause the blade 1102 to move about a central axis into contact with the tether 504.

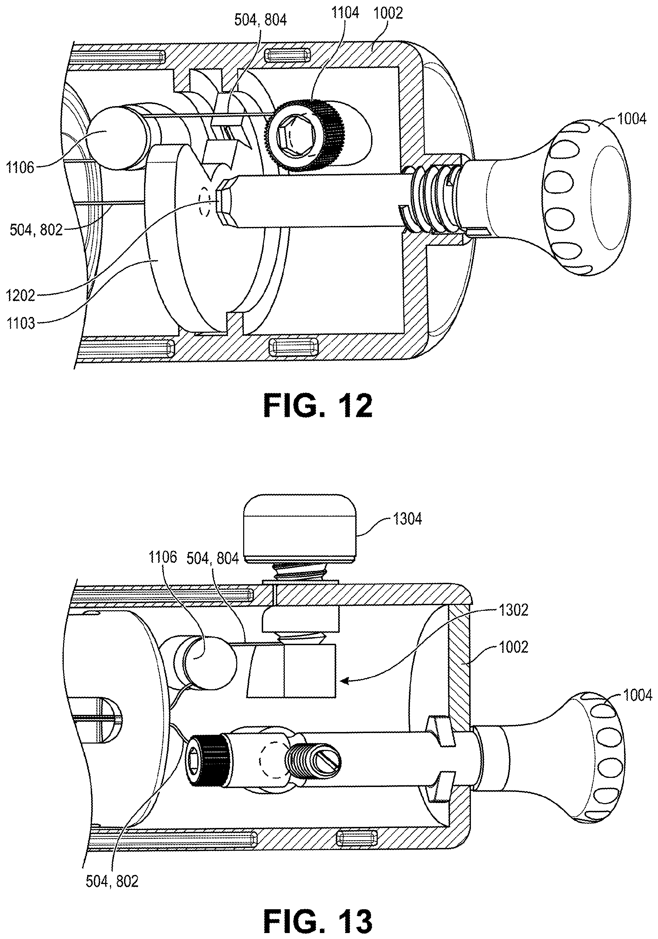

Referring to FIG. 12, a perspective view of a handle of a biostimulator transport system having a cutting wheel is shown in accordance with an embodiment. Rotation of the knob 1004 can drive rotation of the cutting wheel 1103. A distal end of the knob 1004 can have a knob key 1202 that engages a keyway of the cutting wheel 1103. Accordingly, when the cutting wheel 1103 rotates, e.g., due to rotation of the knob 1004, the blade 1102 can cut the tether 504, e.g., the second tether leg 804, to release the tether 504 from the housing 1002. When the tether 504 is severed, the knob 1004 can be rotated further until the threads on the knob 1004 disengage from the housing 1002. The knob 1004 may then be retracted to pull the tether 504 out of the attachment feature 220 and away from the other components of the biostimulator transport system 300. The biostimulator 100 can therefore be released at the target tissue site.

Referring to FIG. 13, a perspective view of a handle of a biostimulator transport system having a vise is shown in accordance with an embodiment. As described above, one end of the tether 504, e.g., second tether leg 804, can be attached to the handle 304, e.g., housing 1002 via a clamping mechanism, and another end of the tether 504, e.g., first tether leg 802, can be attached to the knob 1004. The tether leg attached to the handle 304 can be released, and the knob 1004 can be removed to retract the tether 504 from the biostimulator 100. In an embodiment, the release of the tether 504 may be by releasing a grip on the tether, rather than by cutting the tether. The handle 304 can include a vise to grip the tether. The vise can have a vise block 1302 extending into the internal space of the housing 1002 from the housing wall, and a clamping mechanism, such as a screw 1304, e.g., a socket head cap screw or a set screw, that moves relative to the vise block 1302. The clamping mechanism can pinch the second tether leg 804 against the vise block 1302 to secure the second tether leg 804 to the housing 1002.

One end of the tether 504 can be wrapped around the post 1106 to reduce the required clamping force, as described above. A user can rotate the screw 1304 to unclamp the tether 504. The knob 1004 can be keyed such that rotation of the knob 1004 by a predetermined angle, e.g., 90 degrees, can allow for the proximal removal of the knob 1004 from the housing 1002. Accordingly, when the knob 1004 is unlocked from the housing 1002, the knob can be retracted to pull the tether 504 out of the attachment feature 220 to release the biostimulator 100 at the target tissue site.

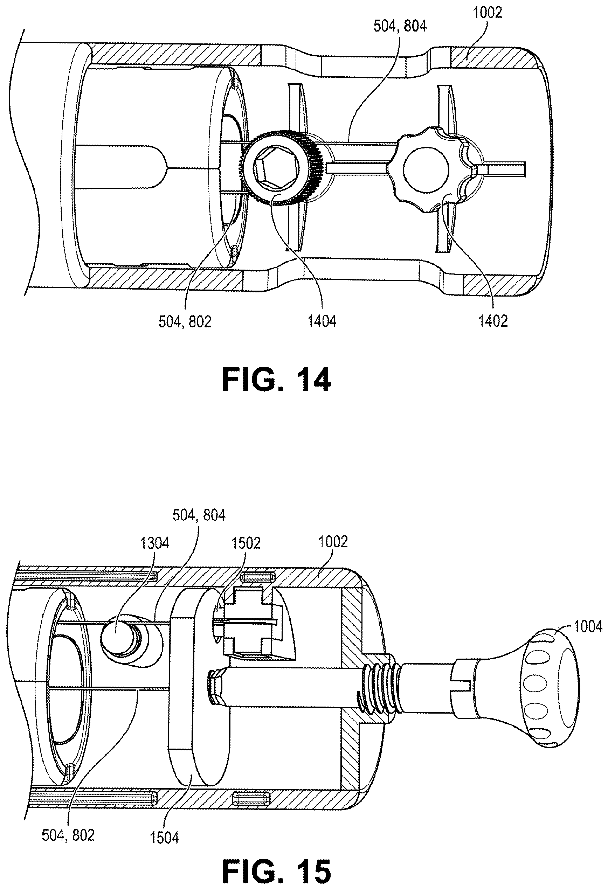

Referring to FIG. 14, a perspective view of a handle of a biostimulator transport system having an open shroud is shown in accordance with an embodiment. The handle 304, e.g., the housing 1002, may have an open shroud design. Similar to the vise embodiment described above, the tether 504, e.g., second tether end 804, may be connected to a clamping mechanism including a knob 1402, and another end of the tether 504, e.g., first tether end 802, can be connected to another clamping mechanism, such as a securing screw 1404. More particularly, one end of the tether 504 can be wrapped about and held by the knob 1402, which may be threaded into the housing 1002, and the other end of the tether 504 may be wrapped and held by the screw 1404. The second tether leg 804 can be released by disengaging the screw 1402, or by cutting the tether between the screws 1402 and 1404. After releasing the second tether leg 804, the tether 504 can be grabbed distal to the screw 1404. The tether leg, e.g., second tether leg 804, can be pulled proximally to remove the tether 504 and release the biostimulator 100.

Referring to FIG. 15, a perspective view of a handle of a biostimulator transport system having a collet is shown in accordance with an embodiment. The handle 304, e.g., the housing 1002 can include a collet 1502 mounted internally. The collet 1502 can include a tapered block that is squeezed by a shuttle 1504. More particularly, the shuttle can be longitudinally movable within the housing 1002, and can have a tapered recess that conforms to jaws of the collet 1502. The shuttle can be advanced distally by rotating the knob 1004 in a first direction. By contrast, rotation of the knob 1004 in an opposite direction can move the shuttle proximally. When the shuttle is moved proximally, and the tapered surface of the recess is drawn against the jaws, the collet 1502 can pinch radially inward. In an embodiment, the second tether leg 804 passes through the jaws. Accordingly, when the shuttle squeezes the jaws inwardly, the jaws can pinch the tether 504, e.g., second tether leg 804. As described above, the first tether leg 802 may pass through the housing 1002 and be connected to the knob 1004.

Rotation of the knob 1004 in a first direction, e.g., clockwise, can drive the shuttle plate forward such that the jaws of the collet open. Whereas the tether 504, e.g., the second tether leg 804, is coupled to the housing 1002 by the collet 1502, when the jaws open, the tether can be released. Rotation of the knob 1004 in a second direction, e.g., counterclockwise, can cause the knob 1004 to unscrew from the housing 1002. Accordingly, the knob 1004 can release from the housing 1002, and a user may pull on the knob 1004 to release the tether 504 from the biostimulator 100.

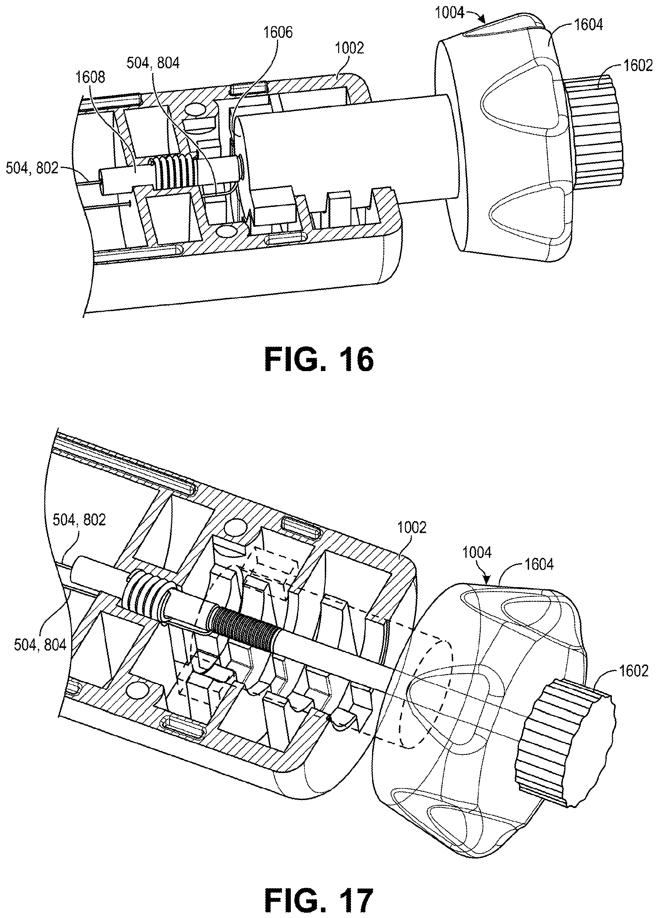

Referring to FIG. 16, a perspective view of a handle of a biostimulator transport system having a twist release is shown in accordance with an embodiment. The knob 1004 can have several components that operate together to provide a twist release mechanism. In an embodiment, the knob 1004 includes an inner knob 1602 threadably coupled to an outer knob 1604. For example, a distal portion of the inner knob 1602 can have a thread that engages a corresponding thread of the outer knob 1604. When the inner knob 1602 is screwed into the outer knob 1604, a distal end of the inner knob 1602 can be flush with or proud of a distal knob end 1606 of the outer knob 1604.

A spring-loaded shaft 1608 can be mounted within the housing 1002, distal to the distal knob end 1606. The spring-loaded shaft 1608 can have a proximal end that opposes the distal knob end 1606 or the distal end of the inner knob 1602. Accordingly, a leg of the tether 504, e.g., the second tether leg 804, can be clamped between the spring-loaded shaft 1608 and the inner knob 1602 or the outer knob 1604. Alternatively, the second tether leg 804 can be looped around the inner knob 1602 at a distal end of the knob that is proud of the distal knob end 1606. The first tether leg 802 can extend through a central lumen of the spring-loaded shaft 1608 to connect to the inner knob 1602 or the outer knob 1604.

Referring to FIG. 17, a perspective view of a handle of a biostimulator transport system having a twist release is shown in accordance with an embodiment. The outer knob 1604 may be releasably coupled to the housing 1002. For example, one or more keys can extend laterally outward from an outer surface of the outer knob 1604. The keys can engage an opposing face of the housing 1002 when the outer knob 1604 is in a first configuration. When the outer knob 1604 is rotated, e.g., 90 degrees, however, the keys can align with a keyway that extends through the housing wall to the surrounding environment. Accordingly, when the outer knob 1604 is rotated to the second configuration, the outer knob 1604 may be free to pass through the slotted keyway out of the housing 1002.

Movement of the outer knob 1604 may be restricted, even when the outer knob 1604 is in the second configuration, by the tether 504. More particularly, when the second tether leg 804 is secured to the outer knob 1604 or the inner knob 1602, tension in the tether 504 may impede the retraction of the knob from the housing 1002. In an embodiment, the inner knob 1602 can be twisted to release the tether 504. More particularly, the tether 504, e.g., second tether leg 804, which may be looped around the inner knob 1602, can be released by rotating the inner knob 1602 such that the distal end of the inner knob 1602 retracts into a body of the outer knob 1604. When the tether 504 is released, the outer knob 1604 can be pushed forward and then rotated from the first configuration to the second configuration. In the second configuration, the keys of the outer knob 1604 can align with the slotted keyway, and thus, the outer knob 1604 may be pulled out of the housing 1002. As the outer knob 1604 is retracted, the released end of the second tether leg 804 will pass through the attachment feature 220 to release the biostimulator 100.

The embodiments above describe tether release mechanisms located within the handle 304. As described below, tether release mechanisms may alternatively be located at a distal end of the biostimulator transport system 300. In each of the embodiments, the biostimulator transport system 300 can include the components described above, e.g., the handle 304, the docking cap, and the torque shaft 602. Additional mechanisms, as described below, may be integrated within the transport system to releasably tether the biostimulator 100 to the biostimulator transport system 300.

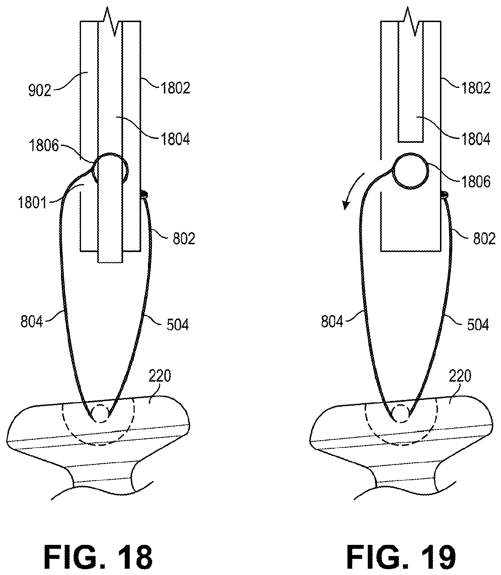

Referring to FIG. 18, a pictorial view of a loop and rod mechanism of a biostimulator transport system is shown in accordance with an embodiment. The biostimulator transport system 300 can include a tubular member 1802. The tubular member 1802 may, for example, be a component of the tether support 502. Accordingly, the tubular member 1802 can have the support lumen 902. In an embodiment, a rod 1804 extends through the support lumen 902. In several embodiments described below, the first tether leg 802 can be coupled to the tubular member 1802, and the second tether leg 804 can be releasably coupled to the rod 1804. More particularly, the rod 1804 can have a first state and a second state. In the first state, the rod 1804 can retain the second tether leg 804, and thus, connect the biostimulator 100 to the transport system. In the second state, the second tether leg 804 is released, and thus, the biostimulator 100 can be released from the transport system.

A first end of the first tether leg 802 can be anchored in place on the tubular member 1802. The first end can be anchored by an adhesive or a mechanical connection. A second end of the tether 504, e.g., an end of the second tether leg 804, can include a loop 1806. More particularly, the tether 504 can extend from the first end at the tubular member 1802 through the gap within the attachment feature 220 to the second end at the loop 1806.

The loop 1806 can be held in place within the tubular member 1802 by a rod 1804. In an embodiment, the tubular member 1802 includes a side port 1801 through a sidewall of the tubular member 1802. The loop 1806 can extend radially through the side port 1801, and the rod 1804 can extend longitudinally through the loop 1806. The rod 1804 can therefore lock the loop 1806 in place within the tubular member 1802 because lateral movement of the loop 1806 is resisted by the rod 1804.

Referring to FIG. 19, a pictorial view of a loop and rod mechanism of a biostimulator transport system is shown in accordance with an embodiment. The rod 1804 can be movable within the tubular member 1802. More particularly, the rod 1804 can be moved from the first state in which the rod 1804 extends through the loop 1806 (FIG. 18) to a second state in which the loop 1806 is distal to the rod 1804. For example, the rod 1804 can be retracted to slide through the loop 1806 until a distal end of the rod 1804 is proximal to the loop 1806.

When the rod 1804 is removed from the loop 1806, the loop may no longer be locked within the tubular member 1802. More particularly, the loop 1806 can move laterally through the side port 1801 to release the tether 504 and to allow it to be removed through the gap in the attachment feature 220. Accordingly, the biostimulator 100 can be released.

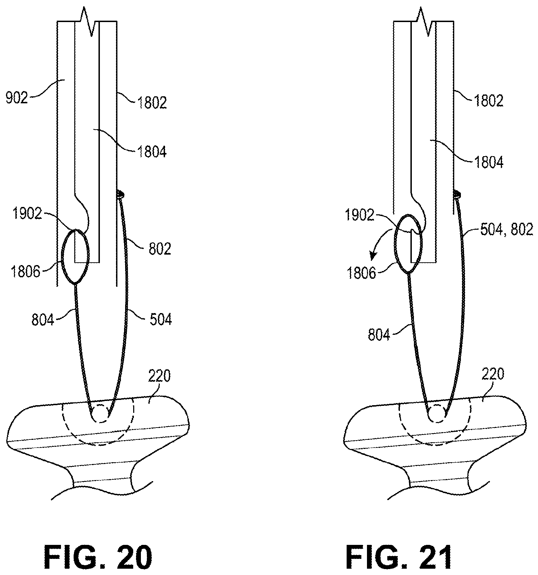

Referring to FIG. 20, a pictorial view of a loop and hook mechanism of a biostimulator transport system is shown in accordance with an embodiment. The loop 1806 can be held in place within the tubular member 1802 by a hook 1902. The hook 1902 can be a feature of the rod 1804. More particularly, the hook 1902 can be integrated in the rod 1804. The rod 1804 can be movable within the tubular member 1802, and in a first state, the rod 1804 can be retracted such that the hook 1902 is located within the tubular member 1802. An outer diameter of the rod 1804 may closely match an inner diameter of the tubular member 1802. Accordingly, in the first state, the loop 1806 may be on the hook 1902, trapped between a recessed surface of the rod 1804 within a crux of the hook 1902 and an inner surface of the tubular member 1802. In the first state, the loop 1806 is retained on the hook 1902, and thus the attachment feature 220 is tethered to the biostimulator transport system 300.

Referring to FIG. 21, a pictorial view of a loop and hook mechanism of a biostimulator transport system is shown in accordance with an embodiment. To release the biostimulator 100, the rod 1804 may be moved from the first state (FIG. 20) in which the loop 1806 is on the hook 1902 to a second state in which the loop 1806 is off the hook 1902. The rod 1804 can be advanced such that the hook 1902 is exposed distally from the tubular member 1802. When the hook 1902 is distal to the tubular member 1802, the loop 1806 may no longer be trapped by the inner surface of the tubular member 1802, and the loop 1806 may therefore fall away from the crux of the hook 1902. When the loop 1806 disengages from the hook 1902, the tubular member 1802 can be retracted to cause the tether 504 and the loop 1806 to pass through the gap in the attachment feature 220. Accordingly, the biostimulator 100 can be released from biostimulator transport system 300.

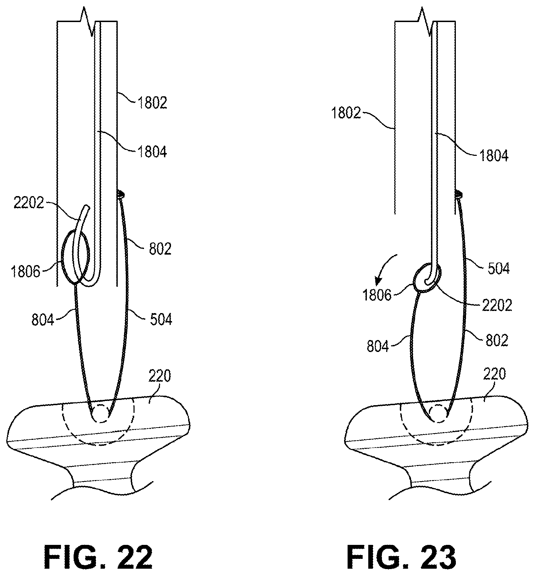

Referring to FIG. 22, a pictorial view of a loop and clasp mechanism of a biostimulator transport system is shown in accordance with an embodiment. The loop 1806 can be held in place within the tubular member 1802 by a deflectable element. For example, the rod 1804 can include a deflectable tip 2202. More particularly, the rod 1804 can be formed from a resilient wire, which can move from a bent configuration to a straightened configuration.

In an embodiment, the rod 1804 can be movable within the tubular member 1802, and in a first state, the rod 1804 is bent and retracted such that the deflectable tip 2202 of the rod 1804 forms a clasp that is located within the tubular member 1802. The clasp can pass through the loop 1806 of the tether 504. The deflectable tip 2202 may therefore retain the loop 1806 in the first state, and thus the attachment feature 220 is tethered to the biostimulator transport system 300.

Referring to FIG. 23, a pictorial view of a loop and clasp mechanism of a biostimulator transport system is shown in accordance with an embodiment. To release the biostimulator 100, the rod 1804 may be moved from the first state (FIG. 22) in which the loop 1806 is on the clasp formed by the deflectable tip 2202 of the rod 1804, to a second state in which the rod 1804 straightens and the loop 1806 is able to slide off of the deflectable tip 2202. The rod 1804 can be advanced (or the tubular member 1802 can be retracted) such that the deflectable tip 2202 is exposed distally from the tubular member 1802. When the deflectable tip 2202 is distal to the tubular member 1802, the deflectable tip 2202 can resiliently return to a straightened configuration in which it was formed. The loop 1806 may no longer be trapped by the clasp, and the loop 1806 may therefore fall away from the crux of the clasp. When the loop 1806 disengages from the clasp, the tubular member 1802 can be retracted to cause the tether 504 and the loop 1806 to pass through the gap in the attachment feature 220. Accordingly, the biostimulator 100 can be released from biostimulator transport system 300.

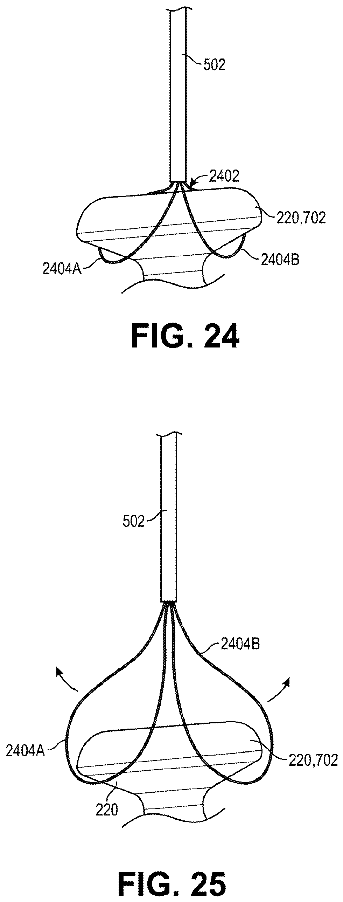

Referring to FIG. 24, a pictorial view of a snare mechanism of a biostimulator transport system is shown in accordance with an embodiment. A tether release mechanism can include a snare 2402. The snare 2402 can extend through the support lumen 902, which is formed in the tether support 502. In an embodiment, the snare 2402 includes several snare loops. For example, a first snare loop 2404A and a second snare loop 2404B can extend distal to the tether support 502. The snare loops can be disposed on the button 702 of the attachment feature 220. More particularly, the button 702 can have a shape that includes laterally directed knobs, and each snare loop can be looped around a respective knob.

In a first state, as shown in FIG. 24, the snare loops 1806 can be cinched onto the attachment feature 220 by positioning the tether support 502 adjacent to the attachment feature 220. More particularly, when the tether support 502 is slid distally over the snare 2402, the snare loops can reduce in size to cinch onto the button 702. The tether 504 may therefore hold the attachment feature 220 and connect the biostimulator 100 to the biostimulator transport system 300.

Referring to FIG. 25, a pictorial view of a snare mechanism of a biostimulator transport system is shown in accordance with an embodiment. In a second state, the tether support 502 can be retracted to loosen the snare 2402. As the tether support 502 is moved proximally, the snare loops can increase in size. The larger loops may no longer hold the button 702 tightly, and may move laterally outward to release from the attachment feature 220. When the increased slack in the system allows the snare loops to slide off of the attachment feature 220, the biostimulator 100 is released from the biostimulator transport system 300.

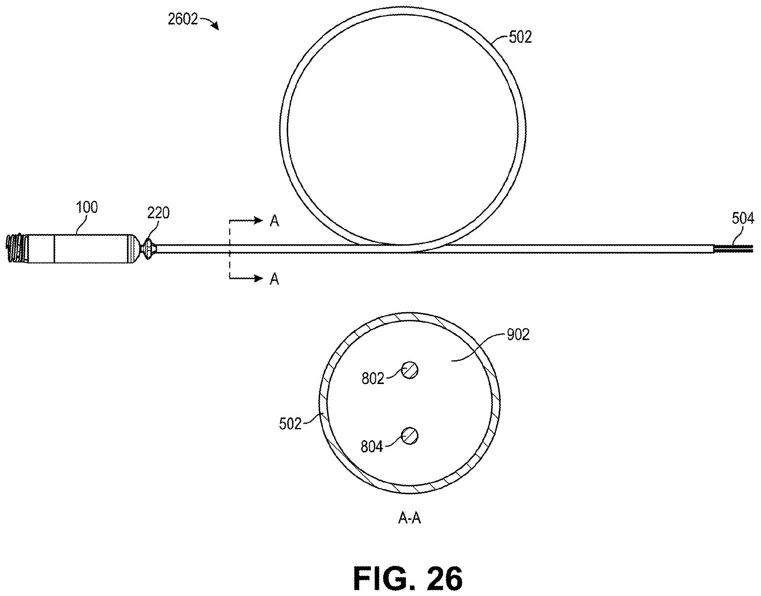

Referring to FIG. 26, is a pictorial view of a loading mechanism for a biostimulator transport system is shown in accordance with an embodiment. A biostimulator 100 that can be packaged separately from the biostimulator transport system 300 can be advantageous. Different types of biostimulators, e.g., types intended for operation at different treatment sites, can be used with a same transport system. Requiring the biostimulators 100 to be packaged with the transport system may, however, take up more shelf space in the catheterization lab. Accordingly, the loading mechanism allows biostimulators to be packaged and stored separately, and loaded into a transport system at the point of care.

In an embodiment, a biostimulator assembly 2602 includes the biostimulator 100, the tether support 502, and the tether 504 integrated as a single unit, separate from the biostimulator transport system 300. The biostimulator 100 has the attachment feature 220, which the tether 504 loops through to form the bight within the gap of the attachment feature 220. The tether legs extend proximally from the bight through the support lumen 902 of the tether support 502.

A cross section A-A of the tether support 502 shows the tether legs within the support lumen 902 of the tether support 502. The tether support 502 may, for example, be formed from a wire coil. The tether support 502 may therefore be a flexible support that provides column strength to the tethers 802, 804.

To load the biostimulator 100 onto the biostimulator transport system 300, a proximal end of the tether support 502 can be inserted through a central lumen of the transport system, e.g., through the docking cap 320 and the torque shaft 602, toward the handle 304. The handle 304 can be adapted to attach to the proximal end of the tether support 502 and the tether legs. For example, the handle 304 can have mechanisms to secure the proximal ends of the tether legs to retain the biostimulator 100 as described above.

The biostimulator assembly 2602 can be loaded into a looped packaging component, e.g., a commonly-termed "racetrack" component, and distributed separately from the biostimulator transport system 300. The biostimulator assembly 2602 may then be unpackaged and loaded into a transport system as needed for treatment of particular target sites.

The biostimulator transport system 300 can incorporate a tether 504 that loops through the attachment feature 220 to retain the biostimulator 100 on the biostimulator transport system 300. For example, the tether 504 can loop through the gap formed by a post or cross beam 704 of the button 702, as described above. In alternative embodiments, the attachment feature 220 may have geometries that receive and retain the tether 504, and which also allow the tether bight 706 to be loaded into the attachment feature 220 without having to pass an entire length (or a portion of the length) of the through the gap. Several such embodiments are described below.

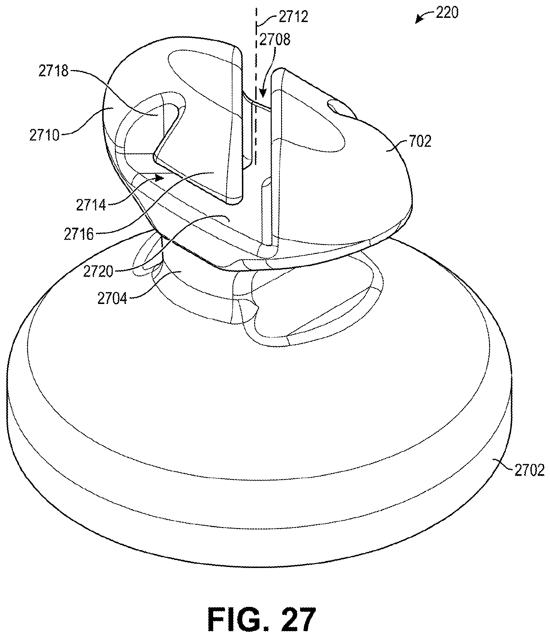

Referring to FIG. 27, a pictorial view of a twist-to-load attachment feature of a leadless biostimulator is shown in accordance with an embodiment. The attachment feature 220 can have a base 2702 that attaches to the biostimulator body. The base 2702 can have an annular wall that connects to the body, and a tapered wall can extend proximally from the base 2702 to a stem 2704. The stem 2704 can extend proximally from the base 2702 to a button 702. Accordingly, the button 702 can extend proximally from the stem 2704 and/or the base 2702.

As described above, the button 702 can engage with the docking cap 320 and provide a mechanism to connect to the tether 504. In an embodiment, the button 702 includes an insertion slot 2708 extending from an outer surface 2710 of the button 702 into the button in a first direction. The first direction can be, for example, along a central axis 2712. The central axis 2712 can be an axis of symmetry of the base 2702. More particularly, the annular wall of the base 2702 can be centered on and extend around the central axis 2712.

The insertion slot 2708 can be sized to receive the tether 504. For example, a portion of the tether 504, e.g., the tether bight 706, can be passed through the insertion slot 2708 by tensioning the tether 504 and then guiding the tether downward through the insertion slot 2708. A width of the insertion slot 2708 can be 1-5 times a width of the tether 504 such that the tether 504 can be passed downward through the insertion slot 2708 when taut, but is unlikely to pass upward through the insertion slot 2708 when slackened.

The insertion slot 2708 may extend along a vertical plane. For example, the insertion slot 2708 can extend vertically through the button 702 along the central axis 2712. The vertical plane may be slanted relative to a symmetry of axis of the button 702. More particularly, when viewed from above, the button 702 may have an elliptical shape symmetric about two perpendicular axes. The insertion slot 2708 can extend along a plane that is oblique to those axes.

In an embodiment, the retention slot 2714 can extend from the insertion slot 2708 in a second direction, e.g., along the horizontal plane, transverse to the first direction, e.g., the direction along the central axis 2712. The retention slot 2714 extends horizontally through the button 702 transverse to the central axis 2712. For example, the retention slot 2714 can be formed along a horizontal plane, and the central axis 2712 may be orthogonal to the horizontal plane. Accordingly, when the tether 504 is inserted through the insertion slot 2708, it can contact a bottom face 2720 defining the retention slot 2714. The tether 504 or button 702 may then be rotated, e.g., pivoted, about the central axis 2712 to slide over the bottom face 2720 and under a tooth 2716 of the button 702. The tooth 2716 can be a portion of the button 702 extending over the bottom face 2720. Accordingly, the retention slot 2714 can be defined between the tooth 2716 and the bottom face 2720.

In an embodiment, the button 702 includes a grip slot 2718 extending upward from the retention slot 2714. The grip slot 2718 can be a vertically extending slot that is offset from the insertion slot 2708. After being rotated through the retention slot 2714, the tether 504 can be lifted upward into the grip slot 2718. When within the grip slot 2718, the tether 504 can be separated from the insertion slot 2708 by the tooth 2716. Accordingly, the tether 504 can be retained within the grip slot 2718 and may be unlikely to fall out of the insertion slot 2708. More particularly, the tether bight 706, which was inserted into the button 702 along the central axis 2712, may be retained within the button 702 until a removal force causes it to slide through the gap in the button 702, as described above.

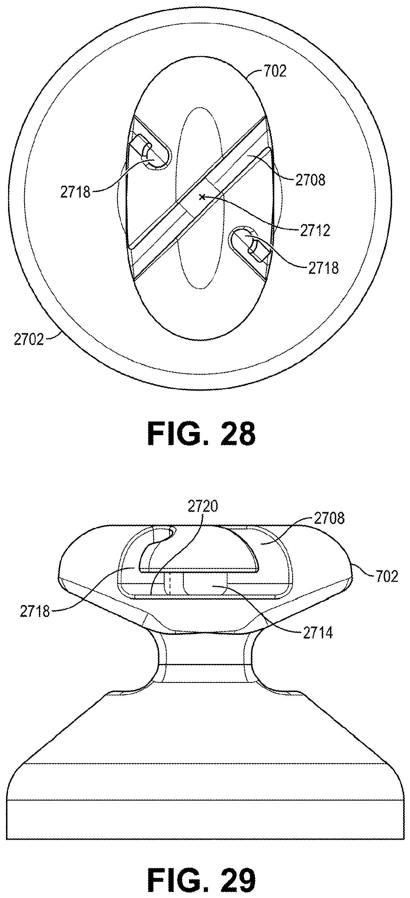

Referring to FIG. 28, a pictorial view of a twist-to-load attachment feature of a leadless biostimulator is shown in accordance with an embodiment. The symmetry of the button 702 is visible when viewed from above. Furthermore, the oblique orientation of the insertion slot 2708 relative to the axis of symmetry of the button 702 is illustrated. Similarly, the grip slot 2718, which extends along an axis oblique or perpendicular to the axis of the insertion slot 2708, is illustrated. It will be appreciated that the tether 504 can be inserted from above along the central axis 2712 through the insertion slot 2708. Such insertion would be in a direction into the page. The tether 504 can then rotated along the retention slot 2714. Rotation may be by rotating the tether 504 clockwise, for example. When the tether 504 is aligned with the grip slots 2718, the tether 504 may be pulled upward. The upward force can pull the tether 504 into the grip slots 2718 to capture and secure the tether bight 706 within the button 702.

Referring to FIG. 29, a pictorial view of a twist-to-load attachment feature of a leadless biostimulator is shown in accordance with an embodiment. In a side view, a lateral separation between the grip slot 2718 and the insertion slot 2708 is visible. The lateral separation is in a horizontal direction in this view, and corresponds to a rotational separation in the view illustrated in FIG. 28. Furthermore, the horizontal extension of the retention slot 2714 between the insertion slot 2708 and the grip slot 2718 is illustrated. More particularly, the retention slot 2714, which is hidden in FIG. 28, is illustrated and providing a horizontal path between the insertion location at the base of the insertion slot 2708 and the capture location at the base of the grip slot 2718. Accordingly, it will be appreciated that the tether 504 can be inserted through the insertion slot 2708 and then moved horizontally, corresponding to the rotation in FIG. 28, from the insertion slot 2708 to the grip slot 2718 to retain the tether 504 within the button 702.

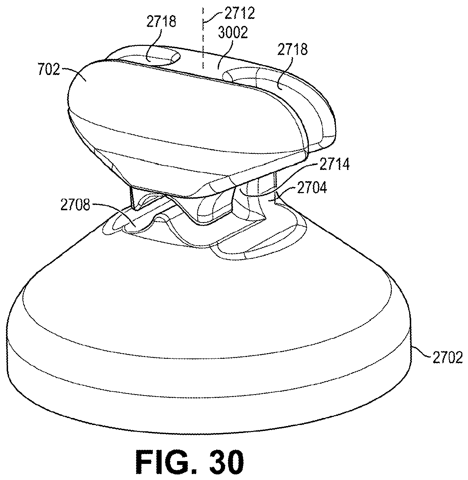

Referring to FIG. 30, a pictorial view of a w-slot attachment feature of a leadless biostimulator is shown in accordance with an embodiment. The w-slot attachment feature can include the base 2702 and the stem 2704 having similar geometries to those components as described above. In an embodiment, the insertion slot 2708, rather than extending vertically through the button 702, can extend horizontally through the button 702. For example, the insertion slot 2708 can extend transverse, e.g., perpendicularly, relative to the vertical central axis 2712.

The insertion slot 2708 may have a profile, which when viewed from the side, can resemble a W. For example, the profile of the insertion slot 2708 can be wave-shaped, undulating, etc. To load the tether bight 706 into the button 702, the tether 504 can be manipulated to take the shape of insertion slot 2708. When properly shaped, the tether 504 can then be moved or is manipulated through the button 702 and/or the stem 2704 toward the central axis 2712. The tether 504 may be unlikely to spontaneously disengage from the insertion slot 2708 because the shape of the insertion slot 2708 can be a shape that the tether 504 does not naturally take.

In an embodiment, the retention slot 2714 extends vertically upward from the insertion slot 2708. More particularly, the retention slot 2714 can extend vertically through the button 702 along the central axis 2712. The button 702 may include a crossbar 3002 at a proximal end of the button 702. The crossbar 3002 can extend horizontally, and the central axis 2712 may extend orthogonal to the crossbar 3002. In an embodiment, the grip slot 2718 can be on either side of the crossbar 3002. Accordingly, after inserting the tether 504 through the insertion slot 2708, the tether 504 can be moved upward through the retention slot 2714, and the tether legs can then be tensioned to cause the tether 504 to cinch against the crossbar 3002 within the respective grip slots 2718. Accordingly, it will be appreciated that the tether 504 can be inserted through the insertion slot 2708 and then moved vertically from the insertion slot 2708 to the grip slot 2718 to retain the tether 504 within the button 702.

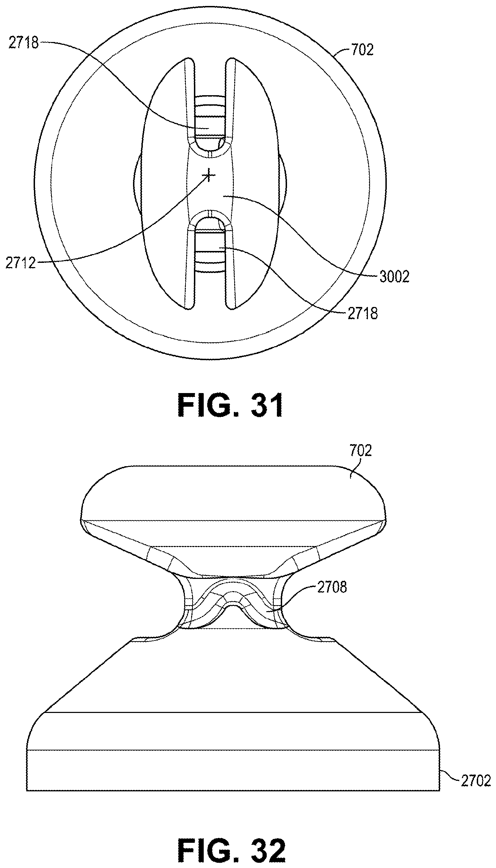

Referring to FIG. 31 a pictorial view of a w-slot attachment feature 220 of a leadless biostimulator 100 is shown in accordance with an embodiment. When viewed from above, the grip slots 2718 on each side of the crossbar 3002 can be seen to extend along a plane that runs parallel to and through the central axis 2712. After being inserted through the insertion slot 2708, the tether 504 can extend essentially through such plane, and may be pulled upward such that the tether legs extend out of the page.

Referring to FIG. 32, a pictorial view of a w-slot attachment feature of a leadless biostimulator is shown in accordance with an embodiment. When viewed from the side, the profile of the insertion slot 2708 can be seen to have a w-shape, or a partial w-shape. More particularly, the profile can undulate and arc above a crest. The slot can have a curvilinear shape that the tether 504 must be manipulated into in order to load tether 504 into insertion slot 2708.

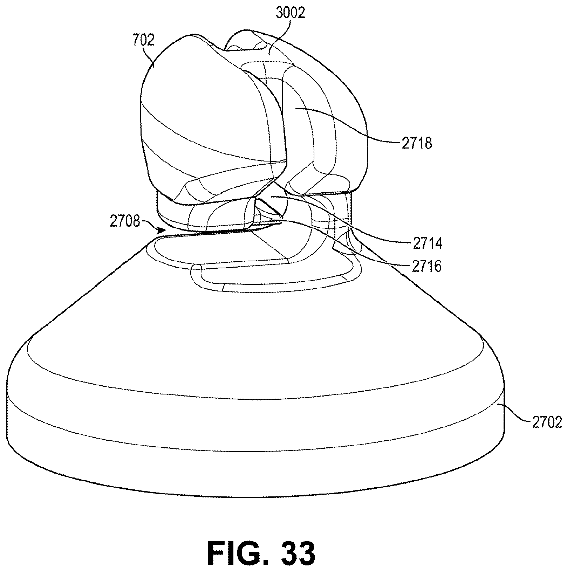

Referring to FIG. 33, a pictorial view of an undercut-slot attachment feature of a leadless biostimulator is shown in accordance with an embodiment. As described above, the insertion slot 2708 can pass horizontally through the stem 2704 and/or the button 702. As with any of the insertion slots 2708 described herein, the slot width may be similar to a width of the tether 504. In an embodiment, the insertion slot 2708 can form an interference fit with the tether 504. More particularly, the slot width may be less than the tether width. Accordingly, a user may press, e.g., floss, the tether 504 through the insertion slot 2708 into the retention slot 2714. The retention slot 2714 may be wider than the tether 504, and thus, the tether 504 may fit within the retention slot 2714 in a slip fit. Accordingly, the tether 504 may be more likely to be retained within the retention slot 2714, than to spontaneously exit through the insertion slot 2708 that has the interference fit.

In an embodiment, the button 702 includes a tooth 2716. The tooth 2716 can extend along a plane parallel to the insertion slot 2708. Furthermore, the tooth 2716 may act as a shelf below the retention slot 2714. Accordingly, the tether 504 can be flossed through the insertion slot 2708 and around the tooth 2716 into the retention slot 2714. Once the tether 504 is located within the retention slot 2714, it may be unlikely to pass around the tooth 2716 back into the insertion slot 2708. The attachment feature 220 can include the grip slots 2718 and the crossbar 3002 as described above with respect to FIGS. 30-32. Accordingly, it will be appreciated that the tether 504 can be inserted through the insertion slot 2708 and then moved vertically from the insertion slot 2708 to the grip slot 2718 to retain the tether 504 within the button 702.

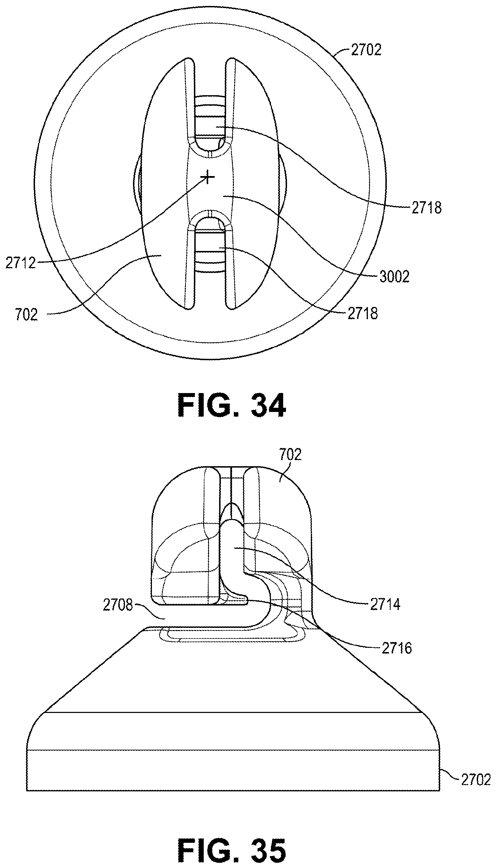

Referring to FIG. 34, a pictorial view of an undercut-slot attachment feature of a leadless biostimulator is shown in accordance with an embodiment. When viewed from above, the grip slots 2718 on each side of the crossbar 3002 can be seen to extend along a plane that runs parallel to and through the central axis 2712. After being inserted through the insertion slot 2708, the tether 504 can extend essentially through such plane, and may be pulled upward such that the tether legs extend out of the page.

Referring to FIG. 35, a pictorial view of an undercut-slot attachment feature of a leadless biostimulator is shown in accordance with an embodiment. When viewed from the side, the profile of the insertion slot 2708 and tooth 2716 can be seen to form a sideways u-shape such that the tether 504 must pass along and around the tooth 2716 in order to access their attention slot. Accordingly, the slot can have a unique shape that the tether 504 must be manipulated into, e.g., via flossing, in order to load the tether 504 into the retention slot 2714 and/or the grip slot 2718.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator transport system (300), comprising:
a handle (304) including a housing (1002);
a docking cap (320) having a docking cavity (404) to receive an attachment feature (220) of a biostimulator (100);
a torque shaft (602) coupled to the docking cap (320);
a tether support (502) extending through the torque shaft (602) to a distal support end (604), wherein the tether support (502) has one or more support lumens (902); and
a tether (504) extending through the one or more support lumens (902) to a tether bight (706) distal to the distal support end (604), **characterized in that**
the handle (304) includes a knob (1004) releasably coupled to the housing (1002); and
the tether (504) is coupled to the knob (1004).

2. The biostimulator transport system of claim 1, wherein the one or more support lumens (902) include a first support lumen (904) separated from a second support lumen (906) by a support septum (908).

3. The biostimulator transport system of claim 2, wherein the tether includes a first tether leg (802) extending proximally from the tether bight (706) through the first support lumen (904) and the second tether leg (804) extending proximally from the tether bight (706) through the second support lumen (906).

4. The biostimulator transport system of claim 1, wherein the tether (504) includes a polymeric filament.

5. The biostimulator transport system of claim 4, wherein the polymeric filament includes one or more of an ultra-high molecular weight polyethylene thread or a polyester thread.

6. The biostimulator transport system of claim 1, wherein the tether (504) includes a metallic filament.

7. The biostimulator transport system of claim 1, wherein the tether (504) includes a first tether leg (802) extending proximally from the tether bight (706) and coupled to the knob (1004), and wherein the tether (504) includes a second tether leg (804) extending proximally from the tether bight (706) and coupled to the housing (1002).

8. The biostimulator transport system of claim 7, wherein the second tether leg (804) is exposed through a window (1006) in the housing (1002).

9. The biostimulator transport system of claim 7, wherein the knob (1004) is rotatably coupled to the housing (1002), and wherein a blade (1102) is mounted on the knob (1004) such that rotation of the knob (1004) causes the blade (1102) to cut the second tether leg (804).

10. The biostimulator transport system of claim 7, wherein the handle (304) includes a vise, and wherein the second tether leg (804) is coupled to the housing (1002) by the vise.

11. The biostimulator transport system of claim 7, wherein the handle (304) includes a collet (1502), and wherein the second tether leg (804) is coupled to the housing (1002) by the collet (1502).

12. The biostimulator transport system of claim 7, wherein the knob (1004) includes an inner knob (1602) threadably coupled to an outer knob (1604).

13. The biostimulator transport system of claim 12, wherein the outer knob (1604) is releasably coupled to the housing (1002), and wherein the second tether leg (804) is clamped between the inner knob (1602) and a spring-loaded shaft (1608).

14. A biostimulator system, comprising a biostimulator (100) mounted on the biostimulator transport system (300) of any one of claims 1-13.

## Patentansprüche

1. Biostimulatortransportsystem (300), das umfasst:
einen Griff (304), der ein Gehäuse (1002) beinhaltet;
eine Andockkappe (320) mit einem Andockhohlraum (404) zur Aufnahme eines Befestigungsmerkmals (220) eines Biostimulators (100);
eine Drehmomentwelle (602), die mit der Andockkappe (320) gekoppelt ist;
eine Bandstütze (502), die sich durch die Drehmomentwelle (602) zu einem distalen Stützende (604) erstreckt, wobei die Bandstütze (502) ein oder mehrere Stützlumen (902) aufweist; und ein Band (504), das sich durch das eine oder die mehreren Stützlumen (902) zu einer Bandschleife (706) erstreckt, die distal des ersten distalen Stützendes (604) liegt, **dadurch gekennzeichnet, dass**
der Griff (304) einen Knauf (1004) beinhaltet, der lösbar mit dem Gehäuse (1002) gekoppelt ist; und
das Band (504) mit dem Knauf (1004) gekoppelt ist.

2. Biostimulatortransportsystem nach Anspruch 1, wobei das eine oder die mehreren Stützlumen (902) ein erstes Stützlumen (904) beinhalten, das durch ein Stütztrennelement (908) von einem zweiten Stützlumen (906) getrennt ist.

3. Biostimulatortransportsystem nach Anspruch 2, wobei das Band einen ersten Bandschenkel (802), der sich proximal von der Bandschleife (706) durch das erste Stützlumen (904) erstreckt, und den zweiten Bandschenkel (804), der sich proximal von der Bandschleife (706) durch das zweite Stützlumen (906) erstreckt, beinhaltet.

4. Biostimulatortransportsystem nach Anspruch 1, wobei das Band (504) ein polymeres Filament beinhaltet.

5. Biostimulatortransportsystem nach Anspruch 4, wobei das polymere Filament eines oder mehrere von einem Faden aus ultrahochmolekularen Polyethylen oder einen Polyesterfaden beinhaltet.

6. Biostimulatortransportsystem nach Anspruch 1, wobei das Band (504) ein metallisches Filament beinhaltet.

7. Biostimulatortransportsystem nach Anspruch 1, wobei das Band (504) einen ersten Bandschenkel (802) beinhaltet, der sich proximal von der Bandschleife (706) erstreckt und mit dem Knauf (1004) gekoppelt ist, und wobei das Band (504) einen zweiten Bandschenkel (804) beinhaltet, der sich proximal von der Bandschleife (706) erstreckt und mit dem Gehäuse (1002) gekoppelt ist.

8. Biostimulatortransportsystem nach Anspruch 7, wobei der zweite Bandschenkel (804) durch ein Fenster (1006) in dem Gehäuse (1002) freigelegt ist.

9. Biostimulatortransportsystem nach Anspruch 7, wobei der Knauf (1004) drehbar mit dem Gehäuse (1002) gekoppelt ist, und wobei eine Klinge (1102) an dem Knauf (1004) befestigt ist, so dass eine Drehung des Knaufs (1004) bewirkt, dass die Klinge (1102) den zweiten Bandschenkel (804) schneidet.

10. Biostimulatortransportsystem nach Anspruch 7, wobei der Griff (304) einen Schraubstock beinhaltet, und wobei der zweite Bandschenkel (804) durch den Schraubstock mit dem Gehäuse (1002) gekoppelt ist.

11. Biostimulatortransportsystem nach Anspruch 7, wobei der Griff (304) eine Spannzange (1502) beinhaltet, und wobei der zweite Bandschenkel (804) durch die Spannzange (1502) mit dem Gehäuse (1002) gekoppelt ist.

12. Biostimulatortransportsystem nach Anspruch 7, wobei der Knauf (1004) einen inneren Knauf (1602) beinhaltet, der mit einem äußeren Knauf (1604) gewindegekoppelt ist.

13. Biostimulatortransportsystem nach Anspruch 12, wobei der äußere Knauf (1604) lösbar mit dem Gehäuse (1002) gekoppelt ist und wobei der zweite Bandschenkel (804) zwischen dem inneren Knauf (1602) und einer federbelasteten Welle (1608) eingeklemmt ist.

14. Biostimulatorsystem, das einen Biostimulator (100) umfasst, der an dem Biostimulatortransportsystem (300) nach einem der Ansprüche 1 bis 13 befestigt ist.

## Revendications

1. Système de transport de biostimulateur (300), comprenant :
une poignée (304) incluant un boîtier (1002) ;
un capuchon d'amarrage (320) ayant une cavité d'amarrage (404) pour recevoir un élément de fixation (220) d'un biostimulateur (100) ;
un arbre de torsion (602) couplé au capuchon d'amarrage (320) ;
un support d'attache (502) s'étendant à travers l'arbre de torsion (602) jusqu'à une extrémité de support distale (604), dans lequel le support d'attache (502) possède une ou plusieurs lumières de support (902) ; et
une attache (504) s'étendant à travers les une ou plusieurs lumières de support (902) jusqu'à une boucle d'attache (706) distale par rapport à l'extrémité de support distale (604),
**caractérisé en ce que**
la poignée (304) inclut un bouton (1004) couplé de manière amovible au boîtier (1002) ; et
l'attache (504) est couplée au bouton (1004).

2. Système de transport de biostimulateur selon la revendication 1, dans lequel les une ou plusieurs lumières de support (902) incluent une première lumière de support (904) séparée d'une seconde lumière de support (906) par un septum de support (908).

3. Système de transport de biostimulateur selon la revendication 2, dans lequel l'attache inclut une première branche d'attache (802) s'étendant de manière proximale à partir de la boucle d'attache (706) à travers la première lumière de support (904) et la seconde branche d'attache (804) s'étendant de manière proximale à partir de la boucle d'attache (706) à travers la seconde lumière de support (906).

4. Système de transport de biostimulateur selon la revendication 1, dans lequel l'attache (504) inclut un filament polymère.

5. Système de transport de biostimulateur selon la revendication 4, dans lequel le filament polymère inclut un ou plusieurs parmi un fil de polyéthylène à poids moléculaire ultra-élevé ou un fil de polyester.

6. Système de transport de biostimulateur selon la revendication 1, dans lequel l'attache (504) inclut un filament métallique.

7. Système de transport de biostimulateur selon la revendication 1, dans lequel l'attache (504) inclut une première branche d'attache (802) s'étendant de manière proximale à partir de la boucle d'attache (706) et couplée au bouton (1004), et dans lequel l'attache (504) inclut une seconde branche d'attache (804) s'étendant de manière proximale à partir de la boucle d'attache (706) et couplée au boîtier (1002).

8. Système de transport de biostimulateur selon la revendication 7, dans lequel la seconde branche d'attache (804) est exposée à travers une fenêtre (1006) dans le boîtier (1002).

9. Système de transport de biostimulateur selon la revendication 7, dans lequel le bouton (1004) est couplé de manière rotative au boîtier (1002), et dans lequel une lame (1102) est montée sur le bouton (1004) de sorte que la rotation du bouton (1004) amène la lame (1102) à couper la seconde branche d'attache (804).

10. Système de transport de biostimulateur selon la revendication 7, dans lequel la poignée (304) inclut un étau, et dans lequel la seconde branche d'attache (804) est couplée au boîtier (1002) par l'étau.

11. Système de transport de biostimulateur selon la revendication 7, dans lequel la poignée (304) inclut une pince de serrage (1502), et dans lequel la seconde branche d'attache (804) est couplée au boîtier (1002) par la pince de serrage (1502).

12. Système de transport de biostimulateur selon la revendication 7, dans lequel le bouton (1004) inclut un bouton interne (1602) couplé par filetage à un bouton externe (1604).

13. Système de transport de biostimulateur selon la revendication 12, dans lequel le bouton externe (1604) est couplé de manière amovible au boîtier (1002), et dans lequel la seconde branche d'attache (804) est serrée entre le bouton interne (1602) et un arbre à ressort (1608).

14. Système de biostimulateur, comprenant un biostimulateur (100) monté sur le système de transport de biostimulateur (300) selon l'une quelconque des revendications 1 à 13.
